# EUROPEAN PATENT APPLICATION

(11) **EP 4 549 053 A1**
(43) Date of publication of application: **07.05.2025**
(21) Application number: 22948827.5
(22) Date of filing: 05.08.2022
(51) Int. Cl.: B22F 1/05, B22F 1/06, B22F 9/22, B01J 31/22, C07C 253/10, C07C 255/04

(54) **MESOPOROUS NICKEL POWDER AND PREPARATION METHOD THEREFOR, NICKEL-PHOSPHORUS CATALYST SYSTEM AND ADIPONITRILE PREPARATION METHOD**

(30) Priority: 01.07.2022 CN 202210775333
(71) Applicant: Zhejiang NHU Company Ltd., Shaoxing, Zhejiang 312500 (CN); Shandong Nhu Fine Chemical Science and Technology Company Ltd., Weifang, Shandong 262737 (CN)
(72) Inventor: CHEN, Zhirong, Hangzhou, Zhejiang 310027 (CN); WU, Wenbin, Shaoxing, Zhejiang 312500 (CN); LIU, Yaqing, Shaoxing, Zhejiang 312500 (CN); MA, Li, Shaoxing, Zhejiang 312500 (CN); YIN, Hong, Hangzhou, Zhejiang 310027 (CN); HUANG, Guodong, Shaoxing, Zhejiang 312500 (CN); XU, Yong, Weifang, Shandong 261108 (CN); ZHANG, Xiongwei, Shaoxing, Zhejiang 312500 (CN)
(74) Representative: Merli, Silvia
(86) International application number: PCT/CN2022/110626
(87) International publication number: WO 2024/000734

(57) **Abstract**

A mesoporous nickel powder and a preparation method therefor, and a nickel-phosphorus catalyst system. The mesoporous nickel powder comprises powder-like particles of metallic nickel, the average particle size of the particles being 1-100 µm, the average pore diameter being 10-60 nm, the pore volume being 0.02-0.09 cm3/g, and the BET specific surface area being 5.0-40.0 m²/g. The preparation method for the mesoporous nickel powder comprises: a hydrothermal synthesis step; a templating agent removal step; and a reduction step; the templating agent is a triblock copolymer comprising an A-B-A structure, or a disulfide bond diblock copolymer comprising an A-S-S-B structure. The nickel-phosphorus catalyst system comprises a complex formed by the mesoporous nickel powder and a phosphorus ligand.

## Description

### TECHNICAL FIELD

The present disclosure relates to a mesoporous nickel powder and a preparation method therefor, and further to a nickel-phosphorus catalyst system for preparing adiponitrile and a preparation method for adiponitrile.

### BACKGROUND

Adiponitrile (ADN), as a major intermediate for organic chemical industry, is mainly used for hydrogenation to produce hexamethylenediamine which is an intermediate for nylon 66 in industry, and can also be used to prepare chemical products such as caprolactam. It is widely applied in such fields as automobiles, engineering plastics, electronic and electrical appliances, precision instruments, and textile industry. Among the available preparation methods for adiponitrile, the butadiene hydrocyanation method has become the most advanced and reasonable adiponitrile production process currently recognized worldwide because of its advantages over other methods, such as accessibility to raw materials, short process route, low cost, low energy consumption, low corrosion to equipment, and high yield. The catalyst used in this method is primarily a catalyst for hydrocyanation of butadiene generated by complexing zero-valent nickel with a phosphorus ligand. However, when coordinated with a phosphorus ligand, nickel powders from different sources have significantly different coordination activities, and the concentrations and activities of the resulting catalysts are also quite different.

CN105033262A discloses a method and system for preparing nickel carbonyl powder from ferronickel powder. The method comprises: bringing finely ground ferronickel powder into contact with CO and a sulfur-containing gaseous compound to afford a first gaseous mixture of nickel carbonyl, iron carbonyl, a sulfur-containing gaseous compound, and CO; condensing the first gaseous mixture to afford a liquid mixture containing nickel carbonyl and iron carbonyl; and decomposing gaseous nickel carbonyl obtained by rectification of the liquid mixture to afford nickel carbonyl powder.

CN108994308A discloses a preparation method for atomized nickel powder with a low apparent density. The method comprises: subjecting an electrolytic nickel plate to melting, deoxidization, atomization when the temperature of the molten liquid reaches 1650°C, and then solid-liquid separation; mixing the nickel powder that has been filtered to dryness with industrial acetic acid; and thereafter drying and reducing the mixture to afford atomized nickel powder with a low apparent density.

CN1827266A discloses a preparation method for nano nickel powder. The method comprises: using a metal nickel plate as an anode, a conductive material as a cathode, and absolute ethanol as an electrolytic solution to carry out an electrochemical reaction in the presence of tetraheptyl ammonium salt TBA as an additive to afford precursor particles of nano nickel; reducing the precursor by heating in ethanol, and then drying by centrifugation to afford nano nickel powder.

CN102909383 discloses a preparation method for ultrafine nickel powder or cobalt powder. The method comprises: crushing and grading the ball-milled precursor of nickel powder or precursor of cobalt powder, then calcining at a temperature of 300°C to 450°C in a reducing atmosphere, and airflow crushing and grading the resultant to afford ultrafine nickel powder or cobalt powder.

When coordinated with a phosphorus ligand, none of the nickel powders prepared by the different preparation processes disclosed in the above-mentioned patent applications exhibits a satisfactory coordination effect. Further, when they are used for preparing catalysts, the mass ratio of the nickel to the phosphorus ligand is low, the resulting catalysts have a low catalytic activity when used in the hydrocyanation reaction, and there is still room for improvement in selectivity to the adiponitrile products.

### SUMMARY

### Technical Problem

In order to solve the above problems existing in the nickel powders currently available on the market, provided are a mesoporous nickel powder having a higher coordination activity with a phosphorus ligand and allowing for a higher mass ratio of the nickel to the phosphorus ligand in the nickel-phosphorus catalyst system, and a preparation method therefor; and further provided are a nickel-phosphorus catalyst system with a higher catalytic activity for use in preparation of adiponitrile, and a preparation method for adiponitrile.

### Solution to Problem

To fulfill the above objective, the present disclosure provides a mesoporous nickel powder, comprising a powder-like particle of metallic nickel, wherein the particle has an average particle size of 1 to 100 µm, preferably 30 to 60 µm, an average pore diameter of 10 to 60 nm, preferably 15 to 40 nm, a pore volume of 0.02 to 0.09 cm³/g, preferably 0.04 to 0.08 cm³/g, and a BET specific surface area of 5.0 to 40.0 m²/g, preferably 10 to 30 m²/g.

The present disclosure further provides a preparation method for the mesoporous nickel powder according to the present disclosure, the method comprising:
a hydrothermal synthesis step of mixing a water-soluble nickel salt, a precipitant, and a template agent in water and heating to obtain a precipitate;
a template agent removal step of removing the template agent from the precipitate by thermal treatment to obtain a precursor of the mesoporous nickel powder; and
a reducing step of reducing the precursor of the mesoporous nickel powder;
wherein the template agent is a triblock copolymer comprising an A-B-A structure or a disulfide bond diblock copolymer comprising an A-S-S-B structure, and the triblock copolymer or the disulfide bond diblock copolymer has a number-average molecular weight of 11000 to 22000;
wherein segment A is a hydrophilic polymer segment, the segment A has a number-average molecular weight of 3000 to 7000, segment B is a hydrophobic polymer segment, and the segment B has a number-average molecular weight of 5000 to 8000; and
the segment A comprises one or more of polyethylene oxide, polyethylene glycol, polypropylene glycol, and polyvinyl alcohol, and the segment B comprises one or more of polyhydroxybutyrate, polycaprolactone, poly(L-lactic acid), and polylactic acid.

The preparation method according to the present disclosure, wherein the template agent comprises one or more of a polyethylene glycol-polyhydroxybutyrate-polyethylene glycol triblock copolymer, a polyethylene glycol-polycaprolactone-polyethylene glycol triblock copolymer, a polyethylene glycol-poly(L-lactic acid)-polyethylene glycol triblock copolymer, and a polyethylene glycol-S-S-poly(L-lactic acid) disulfide bond diblock copolymer.

The preparation method according to the present disclosure, wherein the precipitant comprises one or more of urea, aqueous ammonia, thiourea, hexamethylenetetramine, sodium hydroxide, potassium hydroxide, lithium hydroxide, and barium hydroxide; and the water-soluble nickel salt comprises one or more of nickel nitrate, nickel chloride, nickel bromide, nickel fluoride, nickel iodide, nickel sulfate, nickel acetylacetonate, and nickel acetate tetrahydrate.

The preparation method according to the present disclosure, wherein a molar ratio of the water-soluble nickel salt to the template agent is 1:0.17 to 1:0.25; and a molar ratio of the water-soluble nickel salt to the precipitant is 1: 1 to 1:3.

The preparation method according to the present disclosure, wherein in the hydrothermal synthesis step, the temperature is from 90°C to 120°C, the pressure is from 3.0 to 5.0 MPa, and the time is from 20 to 48 h; in the template agent removal step, the thermal treatment is calcination at 200°C to 800°C for 4 to 8 h; and the reducing step is a step of reducing the precursor of the mesoporous nickel powder at a temperature of 200°C to 600°C for 2 to 8 h in a reducing gas atmosphere; or reducing the precursor of the mesoporous nickel powder for 8 to 24 h in an aqueous solution of a solid reducing agent at a temperature of 80°C to 100°C.

The preparation method according to the present disclosure, wherein reducing gas comprises one or more of hydrogen, methane, ammonia, carbon monoxide, sulfur monoxide, and gaseous sulfur; and the solid reducing agent comprises one or more of sodium borohydride, potassium borohydride, lithium aluminum tetrahydride, lithium borohydride, sodium cyanoborohydride, and thiourea dioxide.

The present disclosure further provides a nickel-phosphorus catalyst system for preparing adiponitrile, comprising a nickel-phosphorus ligand complex formed by the mesoporous nickel powder according to the present disclosure and a phosphorus ligand, or by a mesoporous nickel powder obtained by the preparation method according to the present disclosure and a phosphorus ligand.

The nickel-phosphorus catalyst system according to the present disclosure, wherein a ratio of the mass of the nickel to the mass of the phosphorus ligand in the nickel-phosphorus catalyst system is 1.20% to 1.80%.

The present disclosure further provides a preparation method for adiponitrile, the method comprising: carrying out a hydrocyanation reaction using the catalyst system according to the present disclosure.

### Advantageous Effects of the Invention

According to the mesoporous nickel powder and preparation method therefor provided herein, the mesoporous nickel powder has a higher purity, an average pore diameter of 10 to 60 nm, and a higher coordination activity with phosphorus ligands, and allows for a higher mass ratio of the nickel to the phosphorus ligand in the nickel-phosphorus catalyst system, which may reach 1.30% or more. Besides, the nickel-phosphorus catalyst system futher provided herein formed by the mesoporous nickel powder of the present disclosure and a phosphorus ligand has a higher catalytic activity when used for preparing adiponitrile, and has a higher selectivity to the adiponitrile product, which may be up to 93.1%.

### BRIEF DESCRIPTION OF THE DRAWINGS

FIG. 1 is a 10000-fold magnified SEM image of the nickel powder obtained by atomization in the prior art.
FIG. 2 is a 10000-fold magnified SEM image of the mesoporous nickel powder prepared in Example 1.
FIG. 3 is an XRD pattern of the mesoporous nickel powder prepared in Example 1.
FIG. 4 is a diagram for the particle size distribution of the mesoporous nickel powder prepared in Example 1.

### DETAILED DESCRIPTION

The specific embodiments of the present disclosure will be described in detail below, such that the technical solutions of the present disclosure become apparent.

According to the mesoporous nickel powder provided in the present disclosure, it comprises a powder-like particle of metallic nickel, and the powder-like particle has an average particle size of 1 to 100 µm, preferably 30 to 60 µm; an average pore diameter of 10 to 60 nm, preferably 15 to 40 nm; a pore volume of 0.02 to 0.09 cm³/g, preferably 0.04 to 0.08 cm³/g; and a BET specific surface area of 5.0 to 40.0 m²/g, preferably 10 to 30 m²/g.

According to the mesoporous nickel powder provided in the present disclosure, the mesoporous nickel powder has a higher purity, a large number of porous structures, and a larger specific surface area as compared to various nickel powders provided in the prior art, and the mesoporous nickel powder has a pore size of 10 to 60 nm, which could ensure good entry of sufficient phosphorus ligands into the porous structures, thereby improving the coordination activity of the mesoporous nickel powder.

According to the preparation method for the mesoporous nickel powder provided in the present disclosure, the method comprises:
a hydrothermal synthesis step of mixing a water-soluble nickel salt, a precipitant, and a template agent in water and heating to obtain a precipitate;
a template agent removal step of removing the template agent from the precipitate by thermal treatment to obtain a precursor of the mesoporous nickel powder; and
a reducing step of reducing the precursor of the mesoporous nickel powder;

In the above-mentioned method, the template agent used is a triblock copolymer including an A-B-A structure or a disulfide bond diblock copolymer including an A-S-S-B structure, and the triblock copolymer or the disulfide bond diblock copolymer has a number-average molecular weight of 11000 to 22000. Segment A is a hydrophilic polymer segment and has a number-average molecular weight of 3000 to 7000, and segment B is a hydrophobic polymer segment and has a number-average molecular weight of 5000 to 8000.

In the block copolymer of the template agent, the polymer segment A is a hydrophilic polymer segment mainly including one or more of polyethylene oxide, polyethylene glycol, polypropylene glycol, and polyvinyl alcohol; and the polymer segment B is a hydrophobic polymer segment mainly including one or more of polyhydroxybutyrate, polycaprolactone, poly(L-lactic acid), and polylactic acid.

In a preferred case, the template agent in the present disclosure comprises one or more of a polyethylene glycol-polyhydroxybutyrate-polyethylene glycol triblock copolymer (PEG-PHB-PEG), a polyethylene glycol-polycaprolactone-polyethylene glycol triblock copolymer (PEG-PCL-PEG), a polyethylene glycol-poly(L-lactic acid)-polyethylene glycol triblock copolymer (PEG-PLLA-PEG), and a polyethylene glycol-S-S-poly(L-lactic acid) disulfide bond diblock copolymer (PEG-S-S-PLLA).

In the foregoing description, in the PEG-PHB-PEG, PHB has a number-average molecular weight of 6000 to 7000, and PEG has a molecular weight of 3000 to 4000; in the PEG-PCL-PEG, PEG has a molecular weight of 5000 to 6000, and PCL has a molecular weight of 6000 to 7000; in the PEG-PLLA-PEG, PEG has a molecular weight of 4000 to 5000, and PLLA has a molecular weight of 6000-7000; in the PEG-S-S-PLLA, PEG has a molecular weight of 6000 to 7000, and PLLA has a molecular weight of 7000 to 8000.

Among the preferred block copolymers described above, the PEG-PHB-PEG triblock copolymer (PEG having a molecular weight of 4000 and PHB having a molecular weight of 6000) is more preferred.

The template agents used in the preparation method for the mesoporous nickel powder of the present disclosure are the aforementioned specific block copolymers that are self-assembled during the hydrothermal synthesis reaction to form a highly ordered spatial structure. The nickel hydroxide generated by the hydrothermal synthesis reaction crystallizes and grows on the surfaces of these specific template agents. After removal of the template agents by calcination, mesoporous nickel oxide having a large number of porous structure, a larger specific surface area, and specific ranges of pore size and pore volume is formed as the precursor of the mesoporous nickel powder. The mesoporous nickel oxide is reduced to afford the mesoporous nickel powder with the specific characteristics of the present disclosure.

Compared with homopolymers and random copolymers, the specific block copolymers used as the template agents of the present disclosure have a higher order character of template spatial structure of the template agents, so the pore diameters of the mesoporous nickel powder obtained after removal of the template agent by calcination are more uniform. In addition, the size of the spatial structure formed by the block copolymer in the present disclosure when self-assembled is related to the molecular weight of the block copolymer, so the average pore diameter of the mesoporous nickel powder can be controlled by selecting a specific range of molecular weight of the block copolymer.

The precipitant used in the preparation method for the mesoporous nickel powder of the present disclosure comprises one or more of urea, aqueous ammonia, thiourea, hexamethylenetetramine, sodium hydroxide, potassium hydroxide, lithium hydroxide, and barium hydroxide.

When used as a precipitant, urea as an alkaline substance reacts with an aqueous nickel salt solution during the hydrothermal synthesis reaction to generate a nickel hydroxide precipitate, and the nickel hydroxide is continuously dissolved and precipitated in the aqueous solution under high temperature and high pressure, so as to slowly crystallize and grow on the surfaces of the templating agents; the resulting nickel hydroxide was calcined and reduced to afford the mesoporous nickel powder of the present disclosure. Additionally, similar reactions will also occur in the case of using the other precipitants described above.

The water-soluble nickel salt used in the preparation method for the mesoporous nickel powder of the present disclosure is preferably one or more of nickel nitrate, nickel chloride, nickel bromide, nickel fluoride, nickel iodide, nickel sulfate, nickel acetylacetonate, and nickel acetate tetrahydrate. More preferably, when the water-soluble nickel salt is a composite nickel salt of two or more of the above nickel salts, the complexation activity of the finally obtained mesoporous nickel powder is higher, and the catalytic activity of the catalyst system obtained therefrom is higher.

According to the preparation method for the mesoporous nickel powder provided in the present disclosure, the molar ratio of the water-soluble nickel salt to the template agent is from 1: 0.17 to 1:0.25. When the amount of the template agent is higher than the upper limit of this range, the economic benefit is too low; when the amount of the template agent is lower than the lower limit of this range, the self-assembly effect of the template agent is poor, and the average pore diameter and the total pore volume of the resulting mesoporous nickel powder are too low, resulting in a difficulty in acquisition of the mesoporous nickel powder with the specific characteristics of the present disclosure.

**In** the preparation method of the present disclosure, the molar ratio of the water-soluble nickel salt to the precipitant is from 1:1 to 1:3. When the amount of the precipitant is within this range, the water-soluble nickel salt can be guaranteed to be completely reacted to afford nickel hydroxide. When the amount of the precipitant exceeds the upper limit of this range, the economic benefit is poor; when the amount of the precipitant is less than the lower limit of this range, it is difficult for the water-soluble nickel salt to be fully converted into nickel hydroxide, resulting in a wastage of the water-soluble nickel salt.

According to the preparation method for the mesoporous nickel powder provided in the present disclosure, the hydrothermal synthesis step is carried out at a temperature of 90°C to 120°C and a pressure of 3.0 to 5.0 MPa for 20 to 48 h. Under the high temperature and high pressure as limited above, it is possible to ensure that the solubility of nickel hydroxide in water increases, so that the nickel hydroxide could be repeatedly dissolved and crystallized, and the crystal could grow, so as to ensure the normal progress of the hydrothermal synthesis reaction. Furthermore, the reaction time of 20 to 48 h can guarantee that the hydrothermal synthesis reaction could be conducted adequately, such that the particle size of the nickel hydroxide crystal could grow to the extent where the performance parameters of the resulting mesoporous nickel powder obtained by calcination and reduction are within the specified ranges.

In the template agent removal step of the preparation method of the present disclosure, the precipitate obtained in the hydrothermal synthesis step is filtered, washed, dried, and subjected to thermal treatment, where the thermal treatment is to calcine at a temperature of 200°C to 800°C for 4 to 8 h to afford a precursor of the mesoporous nickel powder, *i.e.,* mesoporous nickel oxide. Thermal treatment carried out under these operating conditions allows for thorough removal of the template agents, and enables the precipitate nickel hydroxide obtained by the hydrothermal synthesis reaction to be fully decomposed to afford the mesoporous nickel oxide.

In the preparation method of the present disclosure, the reducing step is to reduce the above resulting precursor of mesoporous nickel powder, *i.e.,* mesoporous nickel oxide at a temperature of 200°C to 600°C for 2 to 8 h in a reducing gas atmosphere; or to reduce the above resulting mesoporous nickel oxide in an aqueous solution of the solid reducing agent at a reducing temperature of 80°C to 100°C for 8 to 24 h.

In the above reducing step, the reducing gas used comprises one or more of hydrogen, methane, ammonia, carbon monoxide, sulfur monoxide, and gaseous sulfur; the solid reducing agent comprises one or more of sodium borohydride, potassium borohydride, lithium aluminum tetrahydride, lithium borohydride, sodium cyanoborohydride, and thiourea dioxide. Moreover, the molar ratio of the precursor of mesoporous nickel powder (*i.e.*, mesoporous nickel oxide) to the reducing gas or the solid reducing agent is 1: 1.2 to 1:5.

According to the nickel-phosphorus catalyst system for preparing adiponitrile provided in the present disclosure, it comprises a nickel-phosphorus ligand complex formed by the mesoporous nickel powder according to the present disclosure and a phosphorus ligand, or by a mesoporous nickel powder obtained by the preparation method according to the present disclosure and a phosphorus ligand. In a preferred case, the ratio of the mass of the nickel to the mass of the phosphorus ligand in the nickel-phosphorus catalyst system is 1.20% to 1.80%.

In the nickel-phosphorus ligand complex, the phosphorus ligand may be various phosphorus ligands known in the art, including monodentate phosphorus ligands, bidentate phosphorus ligands, tridentate phosphorus ligands, tetradentate phosphorus ligands and phosphorus ligands with a higher denticity value.

For example, the examples of the present application use a monodentate phosphorus ligand represented by the following structural formula 1, a bidentate phosphorus ligand represented by the following structural formula 2, and tetradentate phosphorus ligands represented by the following structural formulae 3 and 4:

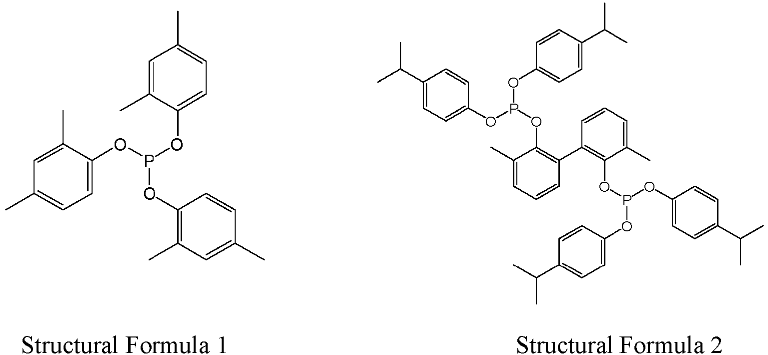

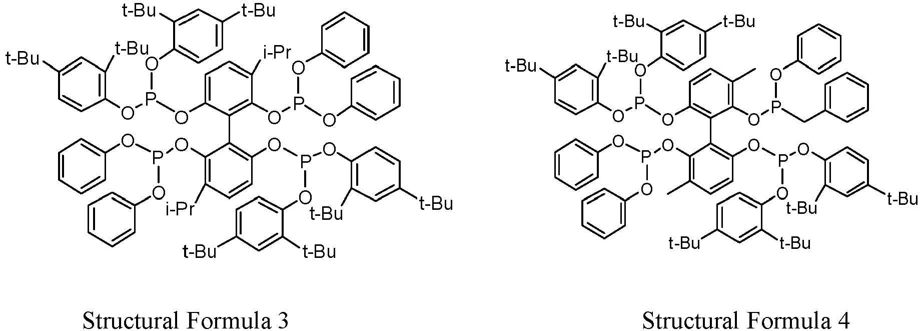

**In** a preferred embodiment, in the case where the mesoporous nickel powder remains unchanged, the complexation activity of the mesoporous nickel powder with the phosphorus ligand gradually increases with the increase of the value of denticity of the phosphorus ligand, so does the catalytic activity of the catalyst system containing a complex formed by the mesoporous nickel powder and the phosphorus ligand; more preferably, the catalyst system comprising a complex formed by a mesoporous nickel powder resulting from a composite nickel salt of two or more water-soluble nickel salts and a phosphorus ligand with a denticity value of three or more has a more excellent catalytic activity.

The aforementioned nickel-phosphorus catalyst system may be prepared by a method known in the art. For example, it is possible to adopt the following method: the mesoporous nickel powder of the present disclosure, a phosphorus ligand, and an auxiliary are dissolved in a nitrile solvent, and subjected to a complexation reaction at a temperature of 60°C to 80°C for 10 to 20 h under the argon protection to afford a nickel-phosphorus catalyst system containing a nickel-phosphorus ligand complex.

In a preferred case, the mass ratio of the nitrile solvent, the phosphorus ligand, the mesoporous nickel powder of the present disclosure, and the auxiliary is (40 to 80):(10 to 30):(2 to 8): 1.

In the aforementioned complexation reaction, the reaction yield of the resulting nickel-phosphorus ligand complex is very high and can be up to 87.21% in the examples of the present disclosure, and the concentration of the nickel can be as high as 6236.5 ppm relative to the solvent in the nickel-phosphorus catalyst system.

In the aforementioned preparation method for the nickel-phosphorus catalyst system, the auxiliary used includes one or more of ferric chloride, ferrous bromide, aluminum chloride, tin tetrachloride, zinc bromide, zinc fluoride, zinc iodide, and zinc chloride. The nitrile solvent used includes one or more of 2-methyl-2-butenenitrile, 2-methyl-3-butenenitrile, 2-pentenenitrile, 3-pentenenitrile, and 4-pentenenitrile; of these, 3-pentenenitrile is preferred.

According to the preparation method for adiponitrile provided in the present disclosure, adiponitrile is prepared by the hydrocyanation reaction, and methods known in the art may be employed. For example, 3-pentenenitrile and HCN are added to the nickel-phosphorus catalyst system of the present disclosure, and reacted under the operating conditions that the temperature is 60°C to 80°C and the pressure is 0.1 to 0.2 MPa.

In the aforementioned preparation method for adiponitrile, the nickel-phosphorus catalyst system provided in the present disclosure is used and it has an excellent catalytic activity, such that the selectivity to the resulting adiponitrile product can be as high as 93.1%.

According to the preparation method for the mesoporous nickel powder provided in the present disclosure, the preferred embodiment comprises the following steps:
(1) a water-soluble nickel salt, a precipitant, and a template agent are added into water at a molar ratio of 1: (1 to 3): (0.17 to 0.25) to formulate 1 mol/L aqueous nickel salt solution, and mixed with stirring at a temperature of 40°C to 60°C till complete dissolution;
(2) the mixed solution obtained in step (1) is transferred into an autoclave lined with polytetrafluoroethylene, and subjected to the hydrothermal synthesis reaction for 20 to 48 h under a autoclave pressure of 3.0 to 5.0 MPa and at a temperature of 90°C to 120°C;
(3) the reaction solution obtained in step (2) is filtered, and the filter cake is washed 2 to 5 times with deionized water and anhydrous ethanol respectively, placed in an oven and dried at 90°C for 6 to 8 h to afford a solid product;
(4) the solid product obtained in step (3) is placed in a tube furnace and calcined at 200°C to 800°C for 4 to 8 h to afford a precursor of mesoporous nickel powder, *i.e.,* mesoporous nickel oxide; and
(5) the mesoporous nickel oxide obtained in step (4) is reduced with a reducing agent (*e.g.*, a reducing gas or a solid reducing agent) to afford the mesoporous nickel powder.

According to the preparation method for the nickel-phosphorus catalyst system for preparing adiponitrile of the present disclosure, a preferred embodiment thereof is as follows: the mesoporous nickel powder obtained in the above step (5) together with a phosphorus ligand and an auxiliary are dissolved into a nitrile solvent, reacted at 60°C to 80°C for 10 to 20 h under the argon protection to afford the nickel-phosphorus catalyst system.

The present disclosure will be described with reference to the examples below, but the present disclosure is by no means limited thereto.

### Example 1: Preparation of Mesoporous Nickel Powder 1

To 100 mL of 1 mol/L aqueous nickel nitrate solution, 0.2 mol of urea was added and stirred until it was completely dissolved. Subsequently, 0.02 mol of PEG-PHB-PEG triblock copolymer (in which the molecular weight of PEG was 4000 and the molecular weight of PHB was 6000) as a template agent was added, and stirred at 50°C until it was completely dissolved.

The resulting mixed solution was transferred into an autoclave lined with polytetrafluoroethylene, and subjected to the hydrothermal synthesis reaction under stirring for 24 h. The pressure in the autoclave was 4.0 MPa and the reaction temperature was 100°C.

After the hydrothermal synthesis reaction was completed, the resulting mixture was filtered, washed three times with deionized water and anhydrous ethanol respectively, and dried in an oven at 90°C for 8 h. The dried powder was calcined at a temperature of 500°C for 6 h to afford mesoporous nickel oxide. The resulting mesoporous nickel oxide was reduced for 6 h at a temperature of 250°C in a hydrogen atmosphere (the molar ratio of hydrogen : mesoporous nickel oxide = 2:1) to afford mesoporous nickel powder 1.

### Determination of Parameters for Mesoporous Nickel Powder

### 1. Average particle size and particle size distribution

Mastersizer 3000 particle size analyzer was used. Test method: (Chinese Pharmacopoeia 2020 Edition, Volume IV: General Principles 0982 Particle Size and Particle Size Distribution - Approach 3 (Light Scattering Method)).

### 2. Purity

The XRD method was adopted. Test method: (Chinese Pharmacopoeia 2020 Edition, Volume IV: General Principles 0451 X-Ray Diffraction).

### 3. BET specific surface area

The nitrogen adsorption method was adopted. Test method: (Evaluation of pore size distribution and porosity of solid materials by mercury porosimetry and gas adsorption - Part 2: Analysis of mesopores and macropores by gas adsorption GB/T 21650.2-2008/ISO 15901-2:2006).

### 4. Average pore diameter

The nitrogen adsorption method was adopted. The test method was the same as described above.

### 5. Pore volume

The nitrogen adsorption method was adopted. The test method was the same as described above.

The mesoporous nickel powder 1 was tested by the above test methods. The results were listed in Table 1 below.

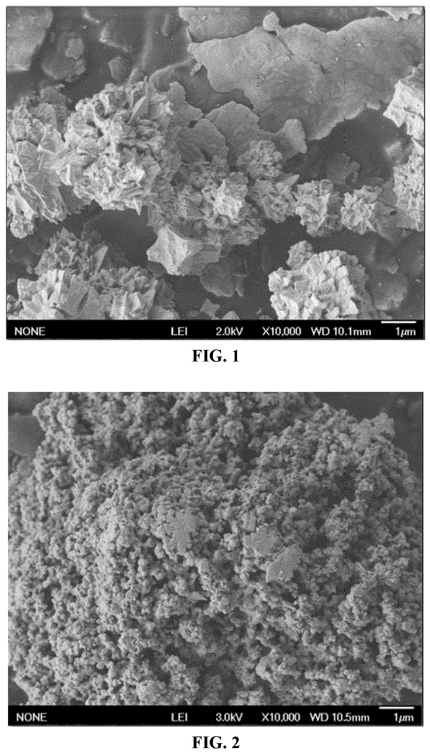

FIG. 2 was a 10000-fold magnified SEM image of the mesoporous nickel powder 1 prepared in Example 1, from which it was evident that the mesoporous nickel powder 1 obtained in Example 1 of the present application had a plenty of porous structures, as compared to the 10000-fold magnified SEM image of the nickel powder obtained by the atomization method disclosed in the prior art CN108994308A.

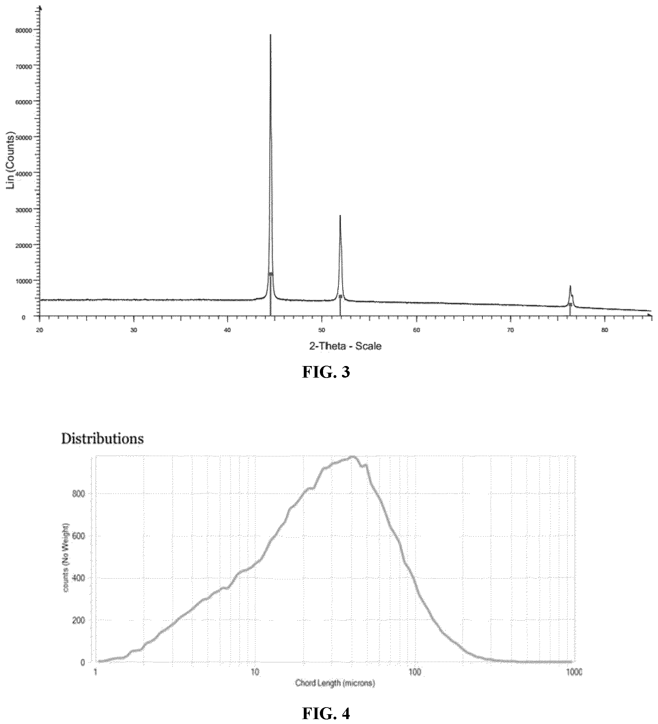

As could be appreciated from the XRD pattern of the mesoporous nickel powder 1 prepared in Example 1 shown in FIG. 3, the nickel oxide was adequately reduced, no impurity peaks occurred, and the purity of the mesoporous nickel powder was high.

As could be appreciated from the diagram for the particle size distribution of the mesoporous nickel powder 1 prepared in Example 1 shown in FIG. 4, the particle size distribution of the mesoporous nickel powder 1 obtained in Example 1 was concentrated, indicating that the particles of the resulting mesoporous nickel powder 1 were uniform.

### Example 2: Preparation of Mesoporous Nickel Powder 2

To 100 mL of 1 mol/L aqueous nickel nitrate solution, 0.2 mol of urea was added and stirred until it was completely dissolved. Subsequently, 0.025 mol of PEG-PCL-PEG triblock copolymer (in which the molecular weight of PEG was 5000 and the molecular weight of PCL was 7000) as a template agent was added, and stirred at 50°C until it was completely dissolved.

The resulting mixed solution was transferred into an autoclave lined with polytetrafluoroethylene, and subjected to the hydrothermal synthesis reaction under stirring for 24 h. The pressure in the autoclave was 4.0 MPa, the rotation speed of the stirrer was 200 r/min, and the reaction temperature was 100°C.

After the hydrothermal synthesis reaction was completed, the resulting mixture was filtered, washed three times with deionized water and anhydrous ethanol respectively, and dried in an oven at 90°C for 8 h. The dried powder was calcined at a temperature of 500°C for 6 h to afford mesoporous nickel oxide. The resulting mesoporous nickel oxide was placed in a 100-mL three-neck flask, to which 50 mL of deionized water was added, and 0.40 mol of potassium borohydride (the molar ratio of potassium borohydride to mesoporous nickel oxide was 4: 1) was added, and stirred to dissolve the potassium borohydride. Thereafter, the reaction solution was reacted at 90°C for 24 h under the argon protection.

Upon completion of the reaction, the reaction solution was transferred into a glove box for filtration. The filter cake was washed three times with anhydrous ethanol and deionized water respectively, and dried at 90°C for 8 h in an argon atmosphere to obtain mesoporous nickel powder 2.

The mesoporous nickel powder 2 was tested by the same test methods described in Example 1. The results were listed in Table 1.

### Examples 3 to 15: Preparation of Mesoporous Nickel Powders 3 to 15

In Examples 3 to 15, mesoporous nickel oxides were prepared by the same method of Example 1, except for altering the types of the template agent, water-soluble nickel salt, and precipitant, the molar ratio of the water-soluble nickel salt, precipitant, and template agent, the reaction temperature, and the reaction time according to Table 1.

In Examples 3, 4, 6, 8, 9, and 11, the mesoporous nickel powders were obtained by the same reduction method for the mesoporous nickel oxide in Example 1. In Examples 5, 7, 10, and 12 to 15, the resulting mesoporous nickel oxides were reduced by the same method of Example 2 to obtain mesoporous nickel powders 3 to 15, except for using the varied types of solid reducing agent as shown in Table 1.

The mesoporous nickel powders were tested by the same test methods described in Example 1. The results were listed in Table 1.

### Comparative Examples 1 to 3: Preparation of Mesoporous Nickel Powders 16 to 18

Mesoporous nickel powders 16 and 17 were prepared by the same method of Example 1, except for changing the template agent to P123 (polyethylene oxide-polypropylene oxide-polyethylene oxide (PEO-PPO-PEO) having a molecular weight of 5800), PEG2000, and PEG-PHB-PEG (PHB having a molecular weight of 10000 and PEG having a molecular weight of 8000), respectively.

The mesoporous nickel powders were tested by the same test methods described in Example 1. The results were listed in Table 1.

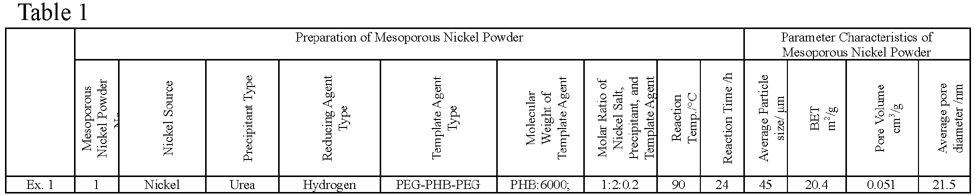

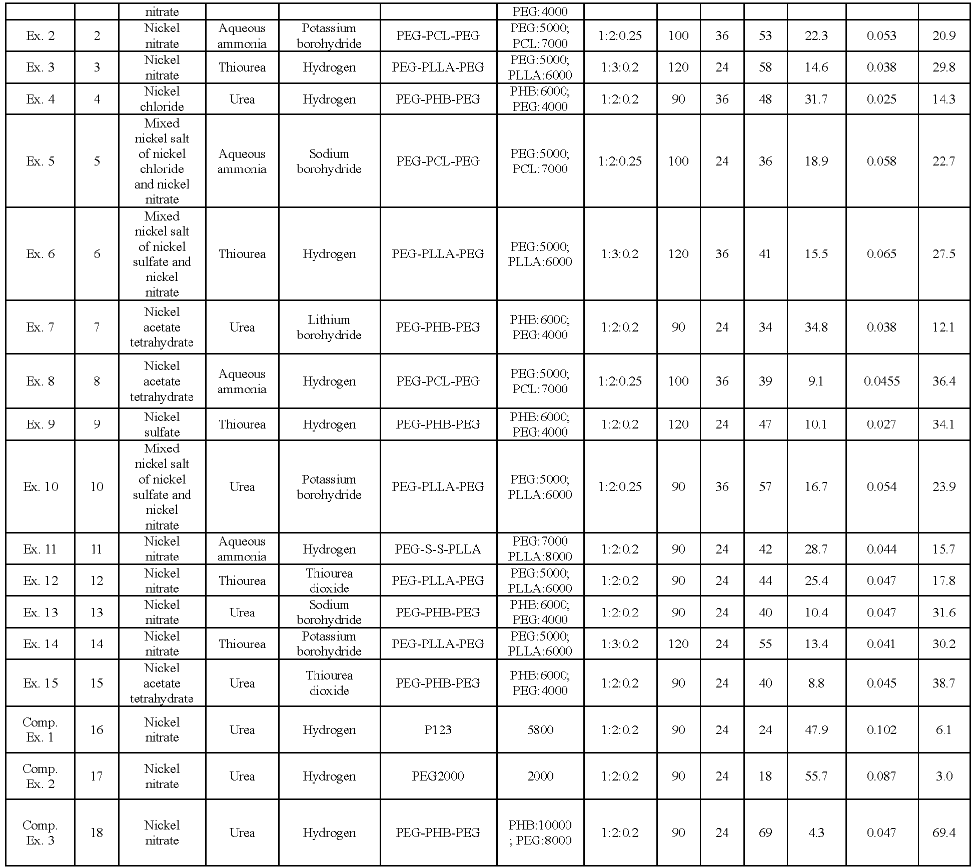

As shown in Table 1, the pore diameters of the mesoporous nickel powders obtained in Examples 1 to 15 of the present disclosure were larger, as compared to Comparative Examples 1 and 2 in which the template agent of the present disclosure was not used; and in Comparative Example 3, all of the number-average molecular weight of the template agent and the number-average molecular weights of segment A and segment B were greater than the ranges set forth in the present disclosure, so the pore size of its mesoporous nickel powder was too large and beyond the range set forth in the present disclosure.

### Example 16: Preparation of Nickel-Phosphorus Catalyst System

In a glove box, 40.5 g of 3-pentenenitrile, 16.1 g of phosphorus ligand represented by structural formula 3, 2.9 g of mesoporous nickel powder, and 0.68 g of zinc chloride were charged into a 100-mL reaction flask, and reacted at a temperature of 60°C for 10 h under the argon protection to afford a mixed solution including a nickel-phosphorus catalyst system containing a nickel-phosphorus ligand complex. This mixed solution was filtered to remove the unreacted residual nickel powder and insoluble matters, and the resulting filtrate was the nickel-phosphorus catalyst system.

The concentration of nickel was tested by the elemental testing method using an inductively coupled plasma optical emission spectrometer (ICP). The concentration of nickel based on 3-pentenenitrile in the nickel-phosphorus catalyst system was calculated to be 6006.9 ppm (*i.e.*, the tested nickel concentration (based on 3-pentenenitrile) described below).

### Preparation of Adiponitrile using Nickel-Phosphorus Catalyst System

The above nickel-phosphorus catalyst system was diluted with 460 g of 3-pentenenitrile until the nickel concentration (based on 3-pentenenitrile) was 500 ppm. The mixed solution after dilution was heated to 55°C, and 110 g of HCN was pumped into the reaction solution using a metering pump over 4 h to afford a reaction solution containing adiponitrile (ADN), methylglutaronitrile (MGN), and ethylsuccinonitrile (ESN).

The resulting reaction solution was tested with GC, and three kinds of dinitrile, *i.e.,* MGN, ESN, and ADN having a mass of 28.2 g, 2.2 g, and 409.6 g respectively were found in the reaction solution.

### Performance Tests

### 1. Yield of nickel-phosphorus ligand complex

Theoretical nickel concentration (based on 3-pentenenitrile) of the resulting nickel-phosphorus ligand complex = mass of phosphorus ligand/molecular weight of phosphorus ligand in the resulting nickel-phosphorus catalyst system*58/(8*(mass of 3-pentenenitrile)/value of denticity of phosphorus ligand).

As calculated, the theoretical nickel concentration based on 3-pentenenitrile was 7151.1 ppm.

Yield of nickel-phosphorus ligand complex = tested nickel concentration (based on 3-pentenenitrile)/theoretical nickel concentration (based on 3-pentenenitrile).

The yield of the nickel-phosphorus ligand complex was calculated to be 84.00% based on the tested nickel concentration and the theoretical nickel concentration.

The yield of the complex could reflect the activity of the mesoporous nickel powder. The higher the yield, the more active the resulting mesoporous nickel powder and the better the effect of the resulting catalyst system for use in the hydrocyanation reaction.

According to the studies of the present inventors, it was found that the pore size of the mesoporous nickel affected the yield of the complex, *i.e.,* the activity of the mesoporous nickel powder.

### 2. Mass ratio of nickel to phosphorus ligand in nickel-phosphorus catalyst system

Mass ratio of nickel to phosphorus ligand in the nickel-phosphorus catalyst system = (nickel concentration in the complexation reaction solution tested by ICP * mass of the complexation reaction solution after removal of excess nickel powder)/mass of phosphorus ligand added during the reaction*100%.

### 3. Selectivity to adiponitrile

Calculation method: In the catalyst system, 3-pentenenitrile reacted with HCN to produce three kinds of dinitrile. As calculated based on the mass and molar amounts of MGN, ESN, and ADN converted from 3-pentenenitrile as tested above, ADN selectivity = molar amount of ADN /(total molar amount of three kinds of dinitrile)*100%.

The selectivity to ADN was calculated to be 93.1%.

### Examples 17 to 33: Preparation of Nickel-Phosphorus Catalyst Systems

The nickel-phosphorus catalyst systems were prepared by the same method of Example 16, except for altering the types of the mesoporous nickel powder, auxiliary, and phosphorus ligand, the mass of the 3-pentenenitrile, phosphorus ligand, mesoporous nickel powder, and auxiliary, the reaction temperature, and the reaction time according to Table 2.

Furthermore, adiponitriles were prepared by the same method of Example 16. The results were listed in Table 2 below.

### Comparative Examples 4 to 10: Preparation of Nickel-Phosphorus Catalyst Systems

In Comparative Examples 3 to 6, the nickel-phosphorus catalyst systems were prepared by the same method of Example 16, except for using mesoporous nickel powders 16, 17, and 18, respectively.

In Comparative Examples 7 to 10, the nickel-phosphorus catalyst systems were prepared by the same method of Example 16, except for using the atomized nickel powder disclosed in CN108994308A, the electrolytic nickel powder disclosed in CN1827266A, the ball-milled nickel powder disclosed in CN102909383, and the evaporated and condensed nickel powder disclosed in CN105033262A.

Furthermore, adiponitriles were prepared by the same method of Example 16 using the aforementioned catalyst systems, respectively. The results were listed in Table 2 below.

**Table 2**

| | Preparation of Nickel-Phosphorus Catalyst System | | | | | | | | Preparation of Adiponitrile | |
|---|---|---|---|---|---|---|---|---|---|---|
| | Nickel Powder Type | Auxiliar y Type | Ligand Type | Mass of 3-Pentenenitrile, Phosphorus Ligand, Nickel Powder, Auxiliary/g | Reaction Temp./°C | Reaction Time/h | Yield of Complex/ % | Mass ratio of Nickel to Phosphorus Ligand in Catalyst System/% | HCN Conversion Rate/% | Selectivity to ADN/% |
| Ex. 16 | Mesoporous nickel powder 1 | ZnCl₂ | Structural formula 3 | 40.5:16.1:2.9:0.68 | 60 | 10 | 84.00 | 1.51 | >99.9 | 93.1 |
| Ex. 17 | Mesoporous nickel powder 2 | AlCl₃ | Structural formula 2 | 40.5:16.3:2.9:0.67 | 70 | 14 | 77.10 | 1.37 | 98.9 | 88.4 |
| Ex. 18 | Mesoporous nickel powder 3 | FeCl₃ | Structural formula 1 | 40.5:15.8:2.9:0.81 | 80 | 20 | 78.30 | 1.44 | 98.6 | 80.3 |
| Ex. 19 | Mesoporous nickel powder 4 | FeCl₃ | Structural formula 3 | 40.5:16.1:2.9:0.81 | 60 | 10 | 76.40 | 1.37 | 98.3 | 92.5 |
| Ex. 20 | Mesoporous nickel powder 5 | ZnCl₂ | Structural formula 3 | 40.5:16.1:2.9:0.68 | 70 | 14 | 86.12 | 1.55 | >99.9 | 93.2 |
| Ex. 21 | Mesoporous nickel powder 6 | ZnCl₂ | Structural formula 3 | 40.5:16.1:2.9:0.68 | 80 | 20 | 86.05 | 1.55 | >99.9 | 93.3 |
| Ex. 22 | Mesoporous nickel powder 7 | FeCl₃ | Structural formula 2 | 40.5:16.3:2.9:0.81 | 60 | 10 | 77.40 | 1.37 | 98.9 | 87.4 |
| Ex. 23 | Mesoporous nickel powder 8 | AlCl₃ | Structural formula 1 | 40.5:15.8:2.9:0.67 | 70 | 14 | 77.90 | 1.43 | 98.3 | 80.7 |
| Ex. 24 | Mesoporous nickel powder 9 | AlCl₃ | Structural formula 3 | 40.5:16.1:2.9:0.67 | 80 | 20 | 76.70 | 1.38 | 97.4 | 92.5 |
| Ex. 25 | Mesoporous nickel powder 10 | ZnCl₂ | Structural formula 3 | 40.5:16.1:2.9:0.68 | 60 | 10 | 87.21 | 1.57 | >99.9 | 93.2 |
| Ex. 26 | Mesoporous nickel powder 11 | FeCl₃ | Structural formula 2 | 40.5:16.3:2.9:0.81 | 70 | 14 | 76.20 | 1.35 | 99.4 | 86.6 |
| Ex. 27 | Mesoporous nickel powder 12 | ZnCl₂ | Structural formula 3 | 40.5:16.1:2.9:0.68 | 80 | 20 | 83.70 | 1.51 | >99.9 | 92.4 |
| Ex. 28 | Mesoporous nickel powder 13 | FeCl₃ | Structural formula 2 | 40.5:16.3:2.9:0.81 | 60 | 10 | 78.10 | 1.39 | 99.1 | 87.4 |
| Ex. 29 | Mesoporous nickel powder 14 | AlCl₃ | Structural formula 3 | 40.5:16.1:2.9:0.67 | 80 | 14 | 77.70 | 1.40 | 98.5 | 92.7 |
| Ex. 30 | Mesoporous nickel powder 15 | AlCl₃ | Structural formula 2 | 40.5:16.3:2.9:0.67 | 80 | 20 | 76.80 | 1.36 | 98.1 | 88.0 |
| Ex. 31 | Mesoporous nickel powder 1 | ZnCl₂ | Structural formula 4 | 40.5:15.6:2.9:0.68 | 60 | 12 | 83.15 | 1.54 | >99.9 | 93.0 |
| Ex. 32 | Mesoporous nickel powder 5 | FeCl₃ | Structural formula 4 | 40.5:15.6:2.9:0.81 | 70 | 14 | 79.78 | 1.48 | 98.6 | 93.1 |
| Ex. 33 | Mesoporous nickel powder 11 | ZnCl₂ | Structural formula 4 | 40.5:15.6:2.9:0.68 | 80 | 10 | 84.31 | 1.57 | >99.9 | 92.8 |
| Comp. Ex. 4 | Mesoporous nickel powder 16 | ZnCl₂ | Structural formula 3 | 40.5:16.1:2.9:0.68 | 60 | 10 | 29.27 | 0.53 | 10.3 | 82.4 |
| Comp. Ex. 5 | Mesoporous nickel powder 17 | ZnCl₂ | Structural formula 3 | 40.5:16.1:2.9:0.68 | 60 | 10 | 23.18 | 0.42 | 17.2 | 83.5 |
| Comp. Ex. 6 | Mesoporous nickel powder 18 | ZnCl₂ | Structural formula 3 | 40.5:16.1:2.9:0.68 | 60 | 10 | 57.39 | 1.03 | 45.2 | 88.4 |
| Comp. Ex. 7 | Atomized nickel powder | ZnCl₂ | Structural formula 3 | 40.5:16.1:2.9:0.68 | 60 | 10 | 33.57 | 0.6 | 22.5 | 84.2 |
| Comp. Ex. 8 | Electrolytic nickel powder | ZnCl₂ | Structural formula 3 | 40.5:16.1:2.9:0.68 | 60 | 10 | 34.47 | 0.62 | 30.4 | 83.7 |
| Comp. Ex. 9 | Ball-milled nickel powder | ZnCl₂ | Structural formula 3 | 40.5:16.1:2.9:0.68 | 60 | 10 | 35.54 | 0.64 | 38.4 | 87.4 |
| Comp. Ex. 10 | Evaporated and condensed nickel powder | ZnCl₂ | Structural formula 3 | 40.5:16.1:2.9:0.68 | 60 | 10 | 13.84 | / | <1.0% | <1.0% |

As shown in Table 2, the average pore diameter of the mesoporous nickel powder according to the present disclosure was the major parameter. Where the pore size within the specified range was obtained, the coordination activity of the nickel powder to the phosphorus ligand would not be affected even if the pore volume and the BET specific surface area of the mesoporous nickel powder were smaller values within the ranges of the present disclosure; if the average pore diameter was not in the specified range, it would be impossible to achieve a good coordination effect between the nickel powder and the phosphorus ligand even if both the BET specific surface area and the pore volume were very large.

In addition, all the complexation activities of the mesoporous nickel powders of Examples 1 to 15 used in Examples 16 to 33 of the present disclosure were higher as compared to Comparative Examples 4 to 10, and the yields of the nickel-phosphorus ligand complexes all exceeded 76%. The nickel concentration in the catalyst system containing a nickel-phosphorus ligand complex resulting from the mesoporous nickel powder of the present disclosure was higher, and all the mass ratios of the nickel to the phosphorus ligands in the nickel-phosphorus catalyst systems were 1.30% or more. Furthermore, when the nickel-phosphorus catalyst system of the present disclosure was used for preparing adiponitrile, the HCN conversion rate was greater than 97.0%, and the selectivity to ADN was as high as 93.1%, indicating a higher catalytic activity of the nickel-phosphorus catalyst system of the present disclosure.

**In** all of Examples 16, 19 to 21, 24, 25, 27, 29, and 31 to 33, tetradentate phosphorus ligands were used to form nickel-phosphorus ligand complexes, in which the yields of the complexes were higher, and the complexation activity of these phosphorus ligand was higher. Furthermore, when the catalyst systems obtained from these complexes were used for preparation of ADN by the hydrocyanation reaction, the selectivity to ADN was excellent.

In particular, the mesoporous nickel powders obtained in Examples 5, 6, and 10 as used in Examples 20, 21, 25, and 32 respectively were each formed by using a composite nickel salt of two water-soluble nickel salts, so they exhibited a higher complexation activity as compared to the other mesoporous nickel powders. Moreover, the phosphorus ligands were tetradentate phosphorus ligands, thus the yields of the nickel-phosphorus ligand complexes formed of the mesoporous nickel powders resulting from these composite nickel salts and these tetradentate phosphorus ligands were higher, the mass ratios of the nickel to the phosphorus ligand in the nickel-phosphorus catalyst systems were higher and reached 1.55 or more, and the selectivities to ADN were also higher. As a result, the catalytic activities of the nickel-phosphorus catalyst systems in these examples were more excellent.

## Claims

1. A mesoporous nickel powder, comprising a powder-like particle of metallic nickel, wherein the particle has an average particle size of 1 to 100 µm, an average pore diameter of 10 to 60 nm, a pore volume of 0.02 to 0.09 cm³/g, and a BET specific surface area of 5.0 to 40.0 m²/g.

2. The mesoporous nickel powder according to claim 1, wherein the particle has an average particle size of 30 to 60 µm, an average pore diameter of 15 to 40 nm, a pore volume of 0.04 to 0.08 cm³/g, and a BET specific surface area of 10 to 30 m²/g.

3. A preparation method for the mesoporous nickel powder according to claim 1 or 2, the method comprising:
a hydrothermal synthesis step of mixing a water-soluble nickel salt, a precipitant, and a template agent in water and heating to obtain a precipitate;
a template agent removal step of removing the template agent from the precipitate by thermal treatment to obtain a precursor of the mesoporous nickel powder; and
a reducing step of reducing the precursor of the mesoporous nickel powder;
wherein the template agent is a triblock copolymer comprising an A-B-A structure or a disulfide bond diblock copolymer comprising an A-S-S-B structure, and the triblock copolymer or the disulfide bond diblock copolymer has a number-average molecular weight of 11000 to 22000;
wherein segment A is a hydrophilic polymer segment, the segment A has a number-average molecular weight of 3000 to 7000, segment B is a hydrophobic polymer segment, and the segment B has a number-average molecular weight of 5000 to 8000; and
the segment A comprises one or more of polyethylene oxide, polyethylene glycol, polypropylene glycol, and polyvinyl alcohol, and the segment B comprises one or more of polyhydroxybutyrate, polycaprolactone, poly(L-lactic acid), and polylactic acid.

4. The preparation method according to claim 3, wherein
the template agent comprises one or more of a polyethylene glycol-polyhydroxybutyrate-polyethylene glycol triblock copolymer, a polyethylene glycol-polycaprolactone-polyethylene glycol triblock copolymer, a polyethylene glycol-poly(L-lactic acid)-polyethylene glycol triblock copolymer, and a polyethylene glycol-S-S-poly(L-lactic acid) disulfide bond diblock copolymer.

5. The preparation method according to claim 3, wherein
the precipitant comprises one or more of urea, aqueous ammonia, thiourea, hexamethylenetetramine, sodium hydroxide, potassium hydroxide, lithium hydroxide, and barium hydroxide; and
the water-soluble nickel salt comprises one or more of nickel nitrate, nickel chloride, nickel bromide, nickel fluoride, nickel iodide, nickel sulfate, nickel acetylacetonate, and nickel acetate tetrahydrate.

6. The preparation method according to any one of claims 3 to 5, wherein a molar ratio of the water-soluble nickel salt to the template agent is 1:0.17 to 1:0.25; and a molar ratio of the water-soluble nickel salt to the precipitant is 1: 1 to 1:3.

7. The preparation method according to any one of claims 3 to 6, wherein
in the hydrothermal synthesis step, the temperature is from 90°C to 120°C, the pressure is from 3.0 to 5.0 MPa, and the time is from 20 to 48 h;
in the template agent removal step, the thermal treatment is calcination at 200°C to 800°C for 4 to 8 h; and
the reducing step is a step of reducing the precursor of the mesoporous nickel powder at a temperature of 200°C to 600°C for 2 to 8 h in a reducing gas atmosphere; or reducing the precursor of the mesoporous nickel powder for 8 to 24 h in an aqueous solution of a solid reducing agent at a temperature of 80°C to 100°C.

8. The preparation method according to claim 7, wherein the reducing gas comprises one or more of hydrogen, methane, ammonia, carbon monoxide, sulfur monoxide, and gaseous sulfur; and the solid reducing agent comprises one or more of sodium borohydride, potassium borohydride, lithium aluminum tetrahydride, lithium borohydride, sodium cyanoborohydride, and thiourea dioxide.

9. A nickel-phosphorus catalyst system for preparing adiponitrile, comprising a nickel-phosphorus ligand complex formed by the mesoporous nickel powder according to claim 1 or 2 and a phosphorus ligand, or by a mesoporous nickel powder obtained by the preparation method according to any one of claims 3 to 8 and a phosphorus ligand.

10. The nickel-phosphorus catalyst system according to claim 9, wherein a ratio of the mass of the nickel to the mass of the phosphorus ligand in the nickel-phosphorus catalyst system is 1.20% to 1.80%.

11. A preparation method for adiponitrile, the method comprising: carrying out a hydrocyanation reaction using the nickel-phosphorus catalyst system according to claim 9 or 10.
